# FASCICULE DE BREVET EUROPEEN

(11) **EP 2 959 873 B1**
(45) Date de publication et mention de la délivrance du brevet: **14.02.2018**
(21) Numéro de dépôt: 15181660.0
(22) Date de dépôt: 12.12.2012
(51) Int. Cl.: A61F 13/00, A61F 13/02, A61L 15/22, A61L 15/60

(54) **PANSEMENT INTERFACE ADHÉRENT**
HAFTSCHNITTSTELLENBANDAGE
ADHESIVE INTERFACE BANDAGE

(30) Priorité: 19.12.2011 WO PCT/FR2011/053043
(43) Date de publication de la demande: 30.12.2015
(62) Demande divisionnaire de: 12816721.0
(73) Titulaire: Urgo Recherche Innovation et Développement, 21300 Chenove (FR)
(72) Inventeur: LECOMTE, Serge, 213 DIJON (FR); PERNOT, Jean-Marc, 21000 DIJON (FR)
(74) Mandataire: Hubert, Philippe

(56) Documents cités:
- EP-A1- 2 168 607
- WO-A2-00/16725
- FR-A1- 2 936 159

## Description

La présente invention concerne généralement une nouvelle utilisation d'un tricot thermofixé pour la fabrication d'un pansement interface adhérent..

On connaît depuis longtemps le traitement des plaies par des pansements destinés à être mis au contact de la plaie en assurant une interface entre ladite plaie et une compresse absorbante que l'on dépose sur le pansement afin d'absorber les exsudats de la plaie. De tels pansements sont habituellement désignés par l'expression « pansements interface ».

L'ensemble pansement interface plus compresse absorbante est habituellement immobilisé soit à l'aide d'une bande que l'on enroule autour de la partie à soigner, par exemple autour du bras ou de la jambe, soit à l'aide d'un ruban adhésif.

Des pansements interface sont par exemple commercialisés par les sociétés Laboratoires URGO ou Mölnlycke Health Care respectivement sous les dénominations URGOTUL® et Mepitel®.

Ces produits sont généralement constitués d'une armature faite d'un tissu à mailles ouvertes dont les fils sont enrobés d'un gel cohésif, de façon à laisser les mailles essentiellement non obturées.

Diverses études ont montré que le pansement interface URGOTUL® présente des propriétés remarquables en ce qui concerne la favorisation du processus de cicatrisation et en particulier de la prolifération des fibroblastes.

Ces propriétés avantageuses ne semblent pas être le résultat d'un composant particulier mais de la composition globale du gel cohésif, non adhérent et faiblement absorbant qui enrobe les fils du tissu flexible à mailles ouvertes.

Ce gel est formé d'une composition spécifique constituée d'une matrice élastomérique hydrophobe fortement plastifiée et contenant en dispersion une faible quantité de particules hydrophiles d'un hydrocolloïde.

Ce pansement et cette composition spécifique sont décrits dans la demande de brevet WO 00/16725.

Le produit URGOTUL® présente néanmoins l'inconvénient de ne pas adhérer à la peau.

Ainsi, selon la localisation de la plaie sur le corps et en particulier lorsque la zone de la plaie n'est pas plane, une fois posé, ce pansement tombe rapidement avant que le personnel soignant n'ait pu poser la compresse ou fixer de façon définitive l'ensemble interface plus compresse à l'aide d'une bande.

Il serait donc souhaitable de disposer d'un pansement interface présentant les propriétés avantageuses du produit URGOTUL® mais doté d'une adhérence sur la peau supérieure afin d'éviter cet inconvénient.

Diverses solutions ont été proposées pour améliorer les propriétés du produit décrit dans la demande de brevet WO 00/16725.

Ainsi, le document WO 2005/056069 propose de réaliser un pansement interface qui comprend une matrice absorbante. Mais le produit décrit dans ce document reste non adhérent à la peau, et soufre donc des mêmes inconvénients que le produit URGOTUL®.

Pour augmenter l'adhérence d'une matrice absorbante ou non, il peut être envisagé d'incorporer dans cette matrice un additif favorisant l'adhérence tel qu'un produit tackifiant.

L'incorporation d'un produit tackifiant pour augmenter l'adhérence du gel cohésif décrit le document dans WO 00/16725 soulève néanmoins plusieurs difficultés.

L'incorporation de composés tackifiants modifie en effet les propriétés rhéologiques du gel ce qui entraine des difficultés techniques très importantes, voire insurmontables, pour obtenir un enrobage correct des fils du tissu dans des conditions de production industrielle acceptables d'un point de vue économique.

Pour résoudre ces problèmes de fabrication il a notamment été proposé dans le document EP 2 168 607 de fabriquer un produit autoporté en évitant ainsi une étape d'enrobage. Un tel produit est susceptible de présenter une meilleure adhérence grâce à l'obtention d'un pouvoir « happant ».

Cependant, toutes les solutions envisagées jusqu'à ce jour conduisent à reformuler la composition du gel cohésif et par conséquent nécessitent l'utilisation de nouveaux composés.

Il serait souhaitable, afin de ne pas altérer la rentabilité du procédé de fabrication du produit ni modifier ce dernier, en s'assurant ainsi de conserver les remarquables propriétés de cicatrisation des formulations du gel cohésif non adhérent et non absorbant décrit dans le document WO 00/16725, de réaliser un produit adhérent sans modifier la nature des composés utilisés pour réaliser ce gel cohésif.

Ainsi, la présente invention a pour but de fournir un pansement interface plus adhérent que le pansement décrit dans le document WO 00/16725 sans modifier la nature des composés utilisés, ni incorporer de nouveaux composés, dans la formulation du gel cohésif qu'il incorpore.

De façon surprenante, on a trouvé que l'utilisation d'un tissu particulier, à savoir un tricot spécifique, permet de réaliser un pansement interface qui présente une adhérence supérieure à celle du produit décrit dans le document WO 00/16725 et cela sans ajouter ou utiliser de nouveaux composés dans la formulation du gel cohésif qu'il contient.

La présente invention est donc relative à l'utilisation d'un tricot thermofixé à mailles ouvertes avec fils tramés, lesdits fils étant des fils continus à filaments non élastiques, qui présente dans le sens transversal une extensibilité mesurée selon la norme EN 13726-4 comprise entre 0,01 et 0,5 N/cm, comme armature pour la fabrication d'un pansement interface adhérent, ledit pansement comprenant :
- un gel cohésif non adhérent formé d'une matrice élastomérique hydrophobe comprenant :
- pour 100 parties en poids d'élastomère choisi parmi un élastomère tribloc du type styrène - (éthylène - butylène) - styrène ou styrène (éthylène - propylène) - styrène éventuellement associé à un copolymère dibloc du type styrène - (éthylène - butylène) ou styrène - (éthylène-propylène) ;
- 1 000 à 2 000 parties en poids d'une huile de paraffine ;
- de 2 à 20 % en poids, rapporté au poids total de la matrice élastomérique, de particules hydrophiles d'un hydrocolloïde en dispersion ; et - ledit tricot thermofixé, les fils dudit tricot étant enrobés par le gel cohésif non adhérent de façon à laisser les mailles essentiellement non obturées.

Dans le cadre de la présente invention la matrice élastomérique hydrophobe comprend un élastomére choisi parmi les polymères séquencés triblocs du type ABA comportant deux blocs terminaux A styrène et une séquence centrale B qui est une oléfine saturée comme par exemple éthylène - butylène ou éthylène - propylène.

Ces copolymères triblocs peuvent être éventuellement associés à des copolymères diblocs du type AB comportant un bloc A styrène et un bloc B éthylène - propylène ou éthylène - butylène.

Dans le cas d'un mélange de copolymères triblocs ABA et de copolymères diblocs AB , on pourra employer des mélanges de copolymères triblocs ABA et de copolymères diblocs AB commerciaux déjà disponibles ou réaliser des mélanges en toute proportion préalablement choisie à partir de deux produits disponibles indépendamment.

De tels copolymères triblocs à séquence centrale saturée sont bien connus de l'homme de l'art et sont par exemple commercialisés :
- par la société KRATON POLYMERS sous la dénomination KRATON G®, et en particulier sous la dénomination KRATON G1651®, KRATON G1654® ou KRATON G1652® pour les copolymères séquencés poly(styrène-(-éthylène-butylène-)-styrène)(en abrégé SEBS) ;
- par la société KURARAY sous la dénomination SEPTON® pour les copolymères séquencés poly(styrène-(-éthylène-propylène-)-styrène) (en abrégé SEPS).

Comme exemple de mélanges commerciaux de copolymères triblocs et diblocs, on peut citer le produit commercialisé par la société KRATON POLYMERS sous la dénomination KRATON G1657® dont la séquence oléfine est éthylène-butylène.

Comme exemple d'un mélange particulier de copolymères triblocs et diblocs que l'on peut réaliser dans le cadre de la présente invention, on peut citer le mélange :
- d'un SEBS triblocs, comme en particulier le produit commercialisé par la société KRATON POLYMERS sous la dénomination KRATON G1651® ; et
- d'un copolymère diblocs poly(styrène-oléfine) comme en particulier le poly(styrène-éthylène-propylène) commercialisé par la société KRATON POLYMERS sous la dénomination KRATON G1702®.

Dans le cadre de la présente invention, on préférera les copolymères triblocs SEBS ou SEPS ayant une teneur en styrène comprise entre 25 et 45 % en poids par rapport au poids dudit SEBS ou SEPS et présentant un poids moléculaire moyen ou élevé et une viscosité Brookfield au moins égale à 300 cp (mesure faite à 25°C pour une solution à 10 % dans le toluène).

D'une façon encore plus préférée, on utilisera des copolymères séquencés triblocs seuls, de préférence des copolymères triblocs SEBS et en particulier les produits commercialisés par la société KRATON POLYMERS sous les dénominations KRATON G1651® ou KRATON G1654®.

L'élastomère hydrophobe est plastifié par addition d'un élément huileux qui permet d'obtenir un gel fortement cohésif, élastique et d'aspect gras.

Dans le cadre de la présente invention, on choisira de préférence comme élément huileux une huile minérale présentant à la fois une bonne compatibilité avec les élastomères précédemment décrits et une tolérance reconnue vis à vis des tissus de la peau. On utilisera préférentiellement des huiles de paraffine de préférence de faibles viscosités ou des mélanges d'huile de paraffine et de vaseline officinale.

Selon une variante de la présente invention, on pourra aussi utiliser une huile minérale associée à une faible quantité d'huile végétale.

Parmi les huiles plastifiantes convenant particulièrement, on peut citer les produits commercialisés par la société SHELL sous les dénominations ONDINA® et RISELLA® qui sont constitués de mélange à base de composés naphténiques et paraffiniques.

De préférence, on utilisera une huile plastifiante choisie parmi les produits commercialisés sous les dénominations ONDINA 963®, ONDINA 15®, ONDINA 919® et en particulier une huile commercialisée sous les dénominations ONDINA 15® ou ONDINA 919® en association avec une vaseline conforme à la pharmacopée française.

D'une façon générale, la matrice hydrophobe comprend 1 000 à 2 000 parties en poids d'huile de paraffine, de préférence de faible viscosité et 0 à 400 parties en poids de vaseline, pour 100 parties en poids d'élastomère.

Selon un mode de réalisation préférée de l'invention, cette matrice comprendra 100 parties en poids d'élastomère SEBS à poids moléculaire élevé, comme par exemple le KRATON G 1651®, et 1600 parties en poids d'un plastifiant huileux composé de 95 % en poids d'huile de paraffine de faible viscosité et 5 % en poids de vaseline.

Comme indiqué précédemment, la matrice hydrophobe contient en outre en dispersion une faible quantité de particules hydrophiles d'un hydrocolloïde.

Par hydrocolloïde ou particules d'hydrocolloïde, on entend désigner ici tout composé habituellement utilisé par l'homme de l'art pour son aptitude à absorber les liquides aqueux tels que l'eau, le sérum physiologique ou les exsudats d'une plaie.

Comme hydrocolloïdes appropriés, on peut citer par exemple la pectine, les alginates et la carboxyméthylcellulose et ses sels de métal alcalin tels le sodium et le calcium.

Les hydrocolloïdes préférés dans le cadre de la présente invention sont les sels de métal alcalin de la carboxyméthylcellulose, et en particulier la carboxyméthylcellulose de sodium (CMC).

La taille des particules d'hydrocolloïde est avantageusement comprise entre 50 et 100 microns, notamment de l'ordre de 80 microns.

La quantité d'hydrocolloïdes incorporés dans la composition élastomère sera avantageusement de l'ordre de 2 à 20 % en poids, de préférence de 5 à 18 % en poids et de préférence encore de 12 à 16 % en poids, rapportée au poids total de la matrice élastomérique.

La matrice élastomérique peut en outre comprendre, un ou plusieurs agents antioxydants.

Comme exemples d'agents antioxydants appropriés, on peut citer :
- les antioxydants phénoliques comme en particulier les produits commercialisés par la société CIBA SPECIALTY CHEMICALS sous les dénominations IRGANOX 1010®, IRGANOX 565® et IRGANOX 1076®.

Ces antioxydants pourront être utilisés en une quantité de l'ordre de 0,05 à 1 % en poids, de préférence de 0,1 à 0,5 % en poids, rapportée au poids total de la matrice élastomérique.

Dans le cadre de la présente invention, on préférera l'utilisation du produit IRGANOX 1010®.

La matrice élastomérique peut aussi contenir des principes actifs ayant un rôle favorable dans le traitement de la plaie. Ces principes actifs peuvent notamment induire ou accélérer la cicatrisation.

Ces agents actifs pourront être utilisés en une quantité de l'ordre de 0,01 à 20 % en poids, de préférence de 1 à 15 % en poids et en particulier de 2 à 10 % en poids rapportée au poids total de la matrice élastomérique.

Parmi les substances actives susceptibles d'être utilisées dans le cadre de l'invention, on peut citer, à titre d'exemples, des agents antiseptiques, antibiotiques, bactéricides ou bactériostatiques, des agents favorisant la cicatrisation, des agents anti-douleurs ou des agents anti-inflammatoires.

Dans le cadre de la présente invention, l'armature du pansement est un tissu à mailles ouvertes constitué par un tricot spécifique.

En étudiant le retrait d'un pansement interface de la plaie, l'inventeur a constaté que l'adhérence sur la peau dépend non seulement de la nature du gel mais aussi de la nature du tissu sur lequel le gel est enduit.

Il a ainsi été déterminé, de façon tout à fait inattendue, qu'un tricot thermofixé avec fils tramés qui possède un niveau d'extensibilité particulier dans le sens transversal, quand il est soumis à de faibles déformations, permet d'obtenir un produit adhérent sans modifier la nature des constituants du gel cohésif.

La caractéristique essentielle d'un tel tricot est de présenter dans le sens transversal une extensibilité, mesurée selon la norme NF EN 13726-4, comprise entre 0,01 et 0,5 N/cm.

De préférence cette extensibilité sera comprise entre 0,05 à 0,3 N/cm et de préférence encore entre 0,08 à 0,15 N/cm.

Selon une version préférée de la présente invention, ce tricot présentera dans le sens longitudinal une extensibilité mesurée selon la même norme comprise entre 15 et 30 N/cm et de préférence entre 20 et 25 N/cm. Une telle extensibilité dans le sens longitudinal permet en effet un enrobage plus facile et plus régulier du tricot par le gel cohésif du point de vue industriel.

De façon générale, ce tricot est réalisé avec fils tramés, et sera notamment fabriqué selon la technologie dite « mailles jetées ».

De plus, ce tricot est thermofixé.

Cette thermofixation permet de stabiliser sur le plan dimensionnel la structure du tricot après tricotage par effet thermique. Cette opération de thermofixation est couramment utilisée par l'homme de l'art lors de la fabrication d'un tricot dont on veut figer la structure tridimensionnelle. Elle peut être mise en oeuvre à l'aide de différentes technologies soit par passage du tricot dans une série de fours thermorégulés, soit par passage du tricot dans un autoclave, soit encore par passage du tricot entre un ou plusieurs cylindres chauffés. Dans le cadre de la présente invention, on préférera réaliser cette opération de thermofixation par passage du tricot entre 2 cylindres chauffés.

Selon l'invention, ce tricot est réalisé à l'aide de fils continus à filaments non élastiques. Par fil continu à filaments, on entend un fil formé de un ou plusieurs filaments longs retords.

Ce fil sera de préférence choisi parmi les fils de 33 à 115 dtex comprenant 12 à 36 filaments.

Le matériau constitutif des fils est de préférence de nature synthétique et hydrophobe. Ce matériau est avantageusement choisi parmi les polyesters et les polyamides.

Selon la version préférée de l'invention, on utilisera un tricot avec fils tramés thermofixé à base de fils continus en polyester 50 dtex comprenant 24 filaments.

Ce tricot pourra présenter un grammage compris entre 20 à 40 g/m² et de préférence entre 24 à 32 g/m².

Ce tricot pourra présenter des mailles rectangulaires, carrées ou polygonales.

Ces mailles présenteront avantageusement des ouvertures dont la surface unitaire avant enrobage est de l'ordre de 0,5 à 3 mm² et de préférence comprise entre 0,85 et 1,25 mm².

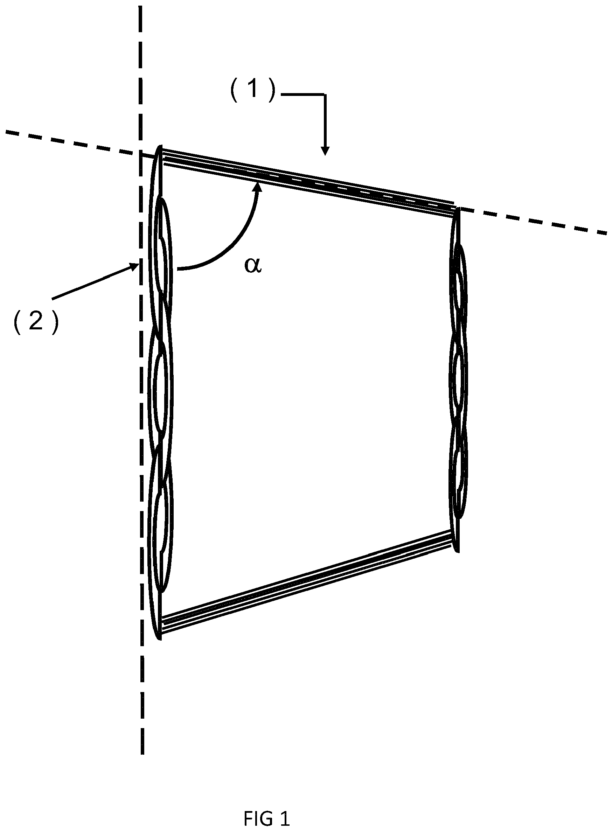

Selon une version préférée de la présente invention, on utilisera un tricot qui présente des mailles trapézoïdales. Un tricot avec une telle maille est illustré de façon schématique sur la figure 1. L'angle α est défini comme l'angle de raccordement moyen des rangées (1) et des colonnes (2) du tricot.

Selon la version préférée de la présente invention, ce tricot présente une surface de maille moyenne de l'ordre de 0,95 mm² et un angle α tel que décrit sur la figue 1 compris entre 75 et 85 degrés.

L'enduction du gel cohésif sur le tricot est réalisée de façon à laisser les mailles essentiellement non obturées, selon les techniques connues de l'homme de l'art et de manière à obtenir une enduction qui varie de 110 à 160 g/m² et de préférence de 125 à 135 g/m².

Dans le cadre de la présente invention, pour réaliser cette enduction on préfèrera mettre en oeuvre le procédé décrit dans la demande de brevet WO 00/16725.

La figure 1 annexée représente de façon schématique une maille du tissu d'un pansement selon un mode de réalisation préféré de l'invention.

L'invention sera illustrée par les exemples et le test comparatif suivants.

### Exemple comparatif :

On a utilisé un pansement interface URGOTUL® comme exemple comparatif. La composition du gel et la réalisation d'un tel pansement est décrite dans l'exemple 1 de la demande de brevet WO 00/16725.

Dans cet exemple comparatif, le tissu était une marquisette thermofixée, en fils de polyester, fabriquée par la société MDB TEXINOV sous la référence 555. Son grammage est de 45 g/m².

Cette marquisette présentait une extensibilité, mesurée selon la norme EN 13726-4, de 2,7 N/cm dans le sens transversal et de 24 N/cm dans le sens longitudinal.

### Exemple selon l'invention :

On a réalisé un pansement selon l'invention selon le même procédé de fabrication et avec la même composition de gel que dans l'exemple 1 de la demande de brevet WO 00/16275 mais en remplaçant la marquisette par un tricot à fils tramés thermofixé, en fils de polyester, fabriqué par la société MDB TEXINOV sous la référence 601. Son grammage était de 28 g/m².

Ce tricot présentait une extensibilité, mesurée selon la norme EN 13726-4, de 0,09 N/cm dans le sens transversal et de 24 N/cm dans le sens longitudinal.

### Mise en évidence de l'adhérence du pansement selon l'invention sur la peau :

Afin d'illustrer l'adhérence supérieure du produit selon l'invention par rapport au produit URGOTUL® on a réalisé un test in vivo comparatif.

Le protocole de ce test était le suivant :
- découper dans un pansement de taille 10 x 10 cm 4 échantillons de format 5 x 5 cm sans retirer les deux films protecteurs,
- prendre un échantillon et enlever un des deux films protecteurs,
- plier l'avant-bras vers le bras (coude en avant),
- appliquer sur le coude avec l'autre main la face de l'échantillon dépourvue de film protecteur en appuyant légèrement,
- enlever le second film protecteur,
- attendre 10 secondes et constater si l'échantillon tombe ou reste en place.

On a nettoyé le coude à l'eau savonneuse, séché avec un mouchoir en papier et recommence l'opération avec un autre échantillon.

Afin d'obtenir des résultats significatifs et reproductibles, 2 échantillons d'un même pansement ont été testés sur le coude gauche et 2 autres échantillons ont été testés sur le coude droit.

On a reproduit ce protocole 10 fois en découpant les échantillons une fois dans le sens longitudinal et une fois dans le sens transversal soit au total 40 échantillons testés.

Une seconde série de tests a été réalisée avec un second testeur soit à nouveau 40 échantillons testés.

Ce test est particulièrement discriminant car le pli du coude est une zone sur laquelle l'adhérence d'un produit est toujours difficile à réaliser. Il illustre aussi les étapes mise en oeuvre par le personnel soignant qui pose le pansement interface.

Les résultats qui ont été obtenus sont rassemblés dans le tableau 1 ci-après.

**Tableau 1**

| Produit | | Selon l'invention | URGOTUL® |
|---|---|---|---|
| | Découpe | Coude gauche | Coude droit |
| Testeur 1 | transversale | 9 /10 | 2 / 10 |
| | longitudinale | 8 / 10 | 1 /10 |
| | | Coude droit | Coude gauche |
| Testeur 2 | transversale | 9 / 10 | 1 / 10 |
| | longitudinale | 10 / 10 | 2 / 10 |

Les résultats du tableau 1 illustrent les avantages du produit fabriqué selon l'utilisation de l'invention par rapport au produit URGOTUL®.

Le produit fabriqué selon l'utilisation de l'invention ne tombe quasiment jamais quel que soit le testeur, le coude testé, le sens de découpe de l'échantillon de pansement et ce résultat est reproductible avec plusieurs pansements. Il n'est tombé que 4 fois sur 40 tests.

A l'inverse, le produit URGOTUL® est tombé quasi systématiquement. Il n'est resté en place que 5 fois sur 40 tests.

Hormis pour 10 % des tests réalisés, ce qui représente les aléas de mesure liés à la nature de la peau du coude ou du testeur et les variabilités sur la fabrication du pansement, on a donc observé une différence de comportement très significative en termes d'adhérence entre le pansement fabriqué selon l'utilisation de l'invention et le produit URGOTUL®.

## Revendications

1. Utilisation d'un tricot thermofixé à mailles ouvertes avec fils tramés, lesdits fils étant des fils continus à filaments non élastiques, qui présente dans le sens transversal une extensibilité mesurée selon la norme EN 13726-4 comprise entre 0,01 et 0,5 N/cm, comme armature pour la fabrication d'un pansement interface adhérent, ledit pansement comprenant :
- un gel cohésif non adhérent formé d'une matrice élastomérique hydrophobe comprenant :
- pour 100 parties en poids d'élastomère choisi parmi un élastomère tribloc du type styrène - (éthylène - butylène) - styrène ou styrène (éthylène - propylène) - styrène éventuellement associé à un copolymère dibloc du type styrène - (éthylène - butylène) ou styrène - (éthylène-propylène) ;
- 1 000 à 2 000 parties en poids d'une huile de paraffine ;
- de 2 à 20 % en poids, rapporté au poids total de la matrice élastomérique, de particules hydrophiles d'un hydrocolloïde en dispersion ; et
- ledit tricot thermofixé, les fils dudit tricot étant enrobés par le gel cohésif non adhérent de façon à laisser les mailles essentiellement non obturées.

2. Utilisation selon la revendication 1, **caractérisé en ce que** les fils constituant le tricot sont choisis parmi des fils de 33 à 115 dtex comprenant 12 à 36 filaments.

3. Utilisation selon l'une des revendications précédentes, **caractérisé en ce que** les fils constituant le tricot sont en polyamide ou en polyester.

4. Utilisation selon l'une des revendications précédentes, **caractérisé en ce que** le tricot présente un grammage compris 20 à 40 g/m² et de préférence entre 24 à 32 g/m².

5. Utilisation selon l'une des revendications précédentes, **caractérisé en ce que** le tricot présente une extensibilité dans le sens longitudinal mesurée selon la norme EN 13726-4 comprise entre 15 et 30 N/cm.

6. Utilisation selon l'une revendications précédentes, **caractérisé en ce que** la matrice élastomérique du gel comprend 1 000 à 2 000 parties en poids d'huile de paraffine et 0 à 400 parties en poids de vaseline officinale pour 100 parties en poids d'un élastomère tribloc de type styrène-(éthylène-butylène)-styrène.

7. Utilisation selon l'une des revendications précédentes, **caractérisé en ce que** l'hydrocolloïde est la carboxyméthylcellulose sodique.

8. Utilisation selon l'une des revendications précédentes, **caractérisé en ce que** l'hydrocolloïde est présent en une quantité de 5 à 18 % en poids rapportée au poids total de la matrice élastomérique.

9. Utilisation selon l'une des revendications précédentes, **caractérisé en ce que** la matrice élastomérique contient en outre un actif.

10. Utilisation selon la revendication 9, **caractérisé en ce que** l'actif est un antiseptique, un antibiotique ou un composé qui favorise la cicatrisation de la plaie.

## Patentansprüche

1. Verwendung eines thermofixierten offenmaschigen Gestricks mit eingeschossenen Fäden, wobei die Fäden Endlosfäden mit unelastischen Filamenten sind, das in Querrichtung eine nach der Norm EN 13726-4 gemessene Dehnbarkeit im Bereich zwischen 0,01 und 0,5 N/cm aufweist, als Armierung für die Herstellung eines Grenzschicht-Klebeverbands, wobei der Verband umfasst:
- ein kohäsives, nicht klebendes Gel, das von einer hydrophoben Elastomermatrix gebildet ist, umfassend:
- für 100 Gewichtsteile Elastomer, ausgewählt aus einem Dreiblock-Elastomer vom Typ Styrol-(Ethylen-Butylen)-Styrol oder Styrol-(Ethylen-Propylen)-Styrol, eventuell mit einem Zweiblock-Copolymer vom Typ Styrol-(Ethylen-Butylen) oder Styrol-(Ethylen-Propylen) assoziiert,
- 1000 bis 2000 Gewichtsteile eines Paraffinöls,
- 2 bis 20 Gew.-%, bezogen auf das Gesamtgewicht der Elastomermatrix, von hydrophilen Partikeln eines Hydrokolloids in Dispersion, und
- das thermofixierte Gestrick, wobei die Fäden des Gestricks mit dem kohäsiven, nicht klebenden Gel umhüllt sind, um die Maschen im Wesentlichen unverschlossen zu lassen.

2. Verwendung nach Anspruch 1, **dadurch gekennzeichnet, dass** die das Gestrick bildenden Fäden aus Fäden mit 33 bis 115 dtex, die 12 bis 36 Filamente umfassen, ausgewählt sind.

3. Verwendung nach einem der vorhergehenden Ansprüche, **dadurch gekennzeichnet, dass** die das Gestrick bildenden Fäden aus Polyamid oder aus Polyester bestehen.

4. Verwendung nach einem der vorhergehenden Ansprüche, **dadurch gekennzeichnet, dass** das Gestrick ein Flächengewicht von 20 bis 40 g/m² und vorzugsweise zwischen 24 und 32 g/m² aufweist.

5. Verwendung nach einem der vorhergehenden Ansprüche, **dadurch gekennzeichnet, dass** das Gestrick eine nach der Norm EN 13726-4 gemessene Dehnbarkeit in Längsrichtung im Bereich zwischen 15 und 30 N/cm aufweist.

6. Verwendung nach einem der vorhergehenden Ansprüche, **dadurch gekennzeichnet, dass** die Elastomermatrix des Gels 1000 bis 2000 Gewichtsteile Paraffinöl und 0 bis 400 Gewichtsteile offizinelle Vaseline für 100 Gewichtsteile eines Dreiblock-Elastomers vom Typ Styrol-(Ethylen-Butylen)-Styrol umfasst.

7. Verwendung nach einem der vorhergehenden Ansprüche, **dadurch gekennzeichnet, dass** das Hydrokolloid Natrium-Carboxymethylcellulose ist.

8. Verwendung nach einem der vorhergehenden Ansprüche, **dadurch gekennzeichnet, dass** das Hydrokolloid in einer Menge von 5 bis 18 Gew.-%, bezogen auf das Gesamtgewicht der Elastomermatrix, vorliegt.

9. Verwendung nach einem der vorhergehenden Ansprüche, **dadurch gekennzeichnet, dass** die Elastomermatrix ferner einen Wirkstoff enthält.

10. Verwendung nach Anspruch 9, **dadurch gekennzeichnet, dass** der Wirkstoff ein Antiseptikum, ein Antibiotikum oder eine Verbindung, welche die Wundheilung der Wunde begünstigt, ist.

## Claims

1. Use of an open-mesh heat-set knit with weft yarns, said yarns being continuous multifilament yarns with non-elastic filaments, which exhibits an extensibility in the transverse direction, measured according to standard EN 13726-4, of between 0.01 and 0.5 N/cm, as a reinforcement in the manufacture of an adherent interface dressing comprising:
- a non-adherent cohesive gel formed from a hydrophobic elastomeric matrix comprising:
- for 100 parts by weight of an elastomer selected from a styrene-(ethylene/butylene)-styrene or styrene-(ethylene/propylene)-styrene triblock elastomer optionally combined with a styrene-(ethylene/butylene) or styrene-(ethylene/propylene) diblock copolymer;
- 1000 to 2000 parts by weight of liquid paraffin; and
- from 2 to 20 % by weight, based on the total weight of the matrix, of hydrophilic particles of a hydrocolloid as a dispersion; and
- said heat-set knit, said yarns of said knit being coated with the non-adherent cohesive gel in such a way as to leave the meshes essentially unsealed.

2. Use as claimed in claim 1, **characterized in that** the yarns constituting the knit are chosen from yarns of 33 to 115 dtex comprising 12 to 36 filaments.

3. Use as claimed in either of the preceding claims, **characterized in that** the yarns constituting the knit are made of polyamide or of polyester.

4. Use as claimed in one of the preceding claims, **characterized in that** the knit has a grammage of between 20 and 40 g/m² and preferably between 24 and 32 g/m².

5. Use as claimed in one of the preceding claims, **characterized in that** the knit exhibits an extensibility in the longitudinal direction, measured according to standard EN 13726-4, of between 15 and 30 N/cm.

6. Use as claimed in one of the preceding claims, **characterized in that** the elastomeric matrix of the gel comprises 1000 to 2000 parts by weight of liquid paraffin and 0 to 400 parts by weight of medicinal petroleum jelly per 100 parts by weight of a triblock elastomer of styrene-(ethylene/butylene)-styrene type.

7. Use as claimed in one of the preceding claims, **characterized in that** the hydrocolloid is sodium carboxymethylcellulose.

8. Use as claimed in one of the preceding claims, **characterized in that** the hydrocolloid is present in an amount of from 5% to 18% of the weight relative to the total weight of the elastomeric matrix.

9. Use as claimed in one of the preceding claims, **characterized in that** the elastomeric matrix also contains an active agent.

10. Use as claimed in claim 9, **characterized in that** the active agent is an antiseptic, an antibiotic or a compound which promotes wound healing.
